# EUROPEAN PATENT APPLICATION

(11) **EP 2 637 119 A2**
(43) Date of publication of application: **11.09.2013**
(21) Application number: 12180360.5
(22) Date of filing: 14.08.2012
(51) Int. Cl.: G06F 19/00, A61B 5/00

(54) **Method, system and apparatus for continuous cardiac personal monitoring**

(30) Priority: 06.03.2012 BR 102012005038
(71) Applicant: Corcam Tecnologia S.A., 04.004.000 São Paulo (BR)
(72) Inventor: Margarida, César Cláudio, 04.006-002 São Paulo (BR); Roriz Júnior, Marcelo de Campos, 04.001-003 São Paulo (BR); Neto, Antonio Andrè, 04.002-031 São Paulo (BR)
(74) Representative: Petraz, Gilberto Luigi

(57) **Abstract**

The present invention relates to a method, a system and an apparatus for remotely monitoring a cardiac condition in an individual in which there is provided a non-invasive, mobile and portable solution including hardware, software, and a back-office application allowing an autonomous and smart remote monitoring. The method may comprise the following steps: Establishing a remote communication between an individual (1) whose cardiac condition is being monitored and a Monitoring Center (2); Establishing a remote communication between a Monitoring Center (2) and a Rescue/Assistance Unit (3); Establishing a remote communication between a Monitoring Center (2) and a Hospital Unit (4); Establishing a remote communication between an individual (1) whose cardiac condition is being monitored and a Medical Staff (5); and Establishing a Data Provision Interface (6) remotely accessible to registered users.

## Description

The present invention relates to a method, a system and an apparatus for remotely monitoring a cardiac condition in an individual in which there is provided a non-invasive, mobile and portable solution including hardware, software, and a back-office application allowing an autonomous and smart remote monitoring.

### BACKGROUND OF THE INVENTION

Cardiovascular diseases are the main cause of death worldwide, arising primarily from the lifestyle and food intake of individuals, as well as from congenital predispositions. Smoking, high cholesterol levels, high blood pressure, engaging in a sedentary lifestyle, and diabetes are factors influencing the occurrence of heart diseases. According to data from the World Health Organization (WHO), heart diseases account for 12% of all deaths.

Heart diseases have worldwide assumed an epidemic status, and despite the advancements in the development of pharmaceuticals, surgical techniques and medical practices, there is still a need for providing new and improved means for preventing cardiac conditions of an individual.

For example, the cardiac condition monitoring of individuals, thus far the best way of prevention, can be performed by regularly visiting the cardiologist, in which electrocardiogram (ECG) comprises the primary means for detecting changes in the cardiac condition of an individual.

The remote monitoring known in the art comprises the use of an equipment attached onto the individual, in which the current ECG monitoring equipments typically remain beside the patient, not allowing for free movement or rendering the remote monitoring difficult. The current options of wireless cardiac monitoring devices generally employ: RF (radio frequency) transmitters and receptors, or communication via Bluetooth (protocol 802.15.1), restricting to a few units the number of equipments simultaneously monitored as well as the distance, which is limited to about 10-20 meters. All currently available options only send data from the patient user to the clinician, thus leaving no possibility of on-demand request; i.e., bilateral.

The state of the art discloses some developments which will be presently incorporated herein by reference.

Patent document CN201898620 relates to a mobile tracker provided with a GSM module (Global System for Mobile Communications) connected to a GPS module unit; a cardioelectric sensor unit is connected to the GSM unit module, and thus the cardioelectric sensor unit detects and collects cardioelectric sensor parameters from the users and transmits them to the GSM module unit. Thus, the GSM module unit transmits positioning messages and cardioelectric parameters through the GPS module unit; therefore, the data from the GSM module unit are transmitted and the GSM module unit is positioned. The mobile tracker can immediately track and locate users equipped with the cardioelectric monitoring equipment when the users are at dangerous cardiac conditions, and in particular can analyze the parameters sent and locate the user having the monitoring equipment.

The patent document above provides the general design for the remote monitoring of the cardiac condition of a patient. The primary elements making up this technique are shown together above in order to provide a minimal system for remote monitoring, however, the equipment sends the parameters of the cardiac function to a base provided remotely, and it is from that base that the parameters are analyzed as being critical or not. It does not disclose teachings regarding the apparatus and its functionalities, apparently suggesting that it works only as cardioelectric data transmitter and collector, besides from setting the user position. The continuous sending of data via GSM, although possible, is costly and requires a data file for each patient being monitored, which requires a much more intricate hardware at the central, which also impacts on the costs. Analyzing the signals sent from the apparatus also demands a time that is critical for the localization, rescue and assistance of the user.

Patent document WO03082093 discloses a system for continuously monitoring a physiological condition of a patient in order to warn the user and a remote service center in case of an abnormality in the physiological condition. To that end, said system comprises monitoring means provided with a set of electrodes arranged on the patient's body to derive a signal related to such condition, the detection means being activated by the electrodes and provided to process the signal in order to derive a characteristic from the signal typical for an abnormality; the warning means being provided to actuate a warning signal by detecting said characteristic from the detection means; the transmission means being provided to transmit the warning signal to a station responsive to the warning signal. The architecture of the system is provided such that only the warning signal is sent to the station, thus enabling it to be continuously monitored.

The patent document described above shows some improvements as compared to the previously disclosed document, however, although the system is able to selectively identify a critical condition of the user and transmit it to a central station, said system does not provide other functionalities, such as indicating the patient location; in addition, the detection and reading of the patient parameters are performed indirectly, which involves the need of interface for collecting and transcripting data for sending a signal suitable to the transmission form.

Patent document US4827943 relates to a multichannel portable physiological data monitoring system for continuously monitoring a patient by means of a continuous connection between a caregiver and a patient which is being monitored that utilizes an intermediate base station and redundant signal paths between the base station and the caregiver. The caregiver wears a unit that receives signals from a base station. The signals from the base station provide information about the individual being monitored and transmit signals used in determining if the patient remains within the range of the base station. The unit worn by the individual that is being monitored may include diagnostics circuits to evaluate signals received from the sensors to transmit a warning signal to the base station when the individual being monitored is in need of assistance. A range monitoring system is provided that will warn the individual being monitored and the caregiver should the patient is moving to an area outside the range of the base station.

The document above teaches about an alternative way of monitoring the condition of an individual, however, in virtue of the hardware employed and the personnel resources, it becomes clearly costly when considered comparatively to the other solutions, yet limited, already shown herein.

Patent document US6287252 relates to a patient monitoring device which comprises an apparatus having an interface operable to receive data signals generated from sensors and a data processor coupled to the interface and operable to format data signals received from the interface at one or more data packets where each packet includes an identifier-transmitter which is unique in the apparatus. The transmitter is included and is operable to receive the structures of the data processor and transmit these structures using radiofrequency signals on a local receptor located proximate to the user. An adhesive placed on the patient has a first surface in which the interface, data processor and the transmitter are provided. An apparatus includes an interface operable to receive data signals generated by the sensors and a data processor coupled to the interface and operable to format data signals received from the interface into one or more data structures where each structure includes an identifier-transmitter which is unique in the apparatus. A transmitter is included and is operable to receive the structures of the data processor and transmit the structures using radiofrequency signals on a local receptor located proximate to the user. The adhesive is placed onto the patient and has a first surface in which the interface, data processor and the transmitter are provided.

The document disclosed above shows a set for monitoring an individual having a higher range than those previously shown. The vital parameters of the patent are collected by sensors and processors attached by mean of adhesives which perform the collection, reading and formatting of data to be transmitted to a remote unit via radiofrequency communication; however, continuously sending reports is costly both due to the data transmission and storage costs. Moreover, the invention provides just a monitoring means without providing any other functionality, such as assistance of the user, localization and interactivity.

Patent document CN101579235 relates to a smart remote ECG monitoring system which relies on an EDGE network comprising a portable ECG signal acquisition end, a network transmission module and a server monitoring terminal in which the portable ECG signal acquisition end is connected to the server monitoring terminal by means of a network transmission module; the network transmission module comprises an EDGE transmission module and a GPS module; and the server monitoring terminal is provided with a transmission daemon and an automatic ECG and diagnostics analysis system. The ECG signal acquisition terminal carries out an acquisition, shows and stores locally an ECG signal being thus the convenient operation; the smart remote ECG monitoring system quickly and efficiently sends and receives data remotely through the EDGE transmission module and performs the warning and GPS positioning functions in emergency case; the automatic ECG analysis und diagnostics system of the server monitoring terminal may independently analyze and diagnose diseases and re-feed the diagnostics information such as to greatly reduce the need of having a medical practitioner present; The chaos-based non-linear dynamic parameter analysis newly adopted improves the comprehension and reliability of the diagnostics.

Said patent document above discloses a set for remotely monitoring a cardiac condition of an individual by reading an ECG having a local storage of data collected and sending the data to a remote station or database file of ECG records, in addition to sending a warning and the location of the individual via GPS and EDGE technologies, however, said system is not directed to the monitoring and identification of ischaemias, and does not offer hands-free communication which provides such an interactivity that it becomes possible to make requests in both ways regardless of the condition of the individual being monitored. Furthermore, it does not have a neural network that allows establishing a customized cardiac profile of the individual, does not provide means for detecting falls nor provides a voice command, which functions significantly improve the monitoring form and the response in critical scenarios.

### OBJECTS OF THE INVENTION

It is, therefore, an object of the present invention to provide a solution to the drawbacks previously discussed.

It is an object of the present invention to provide a method, a system, and an apparatus for remotely monitoring the cardiac condition of an individual.

It is the object of the present invention to provide an apparatus for remotely monitoring the cardiac condition of an individual which is portable, mobile, non-invasive, including hardware, software and a back-office application which allows for the smart and autonomous remote monitoring - via GSM/GPRS, detecting cardiac intercurrences (arrhythmias and ischaemias) through 3 electrodes ('ns I, II and III), falls, as well as integrating hands-free assistance and geographical localization via GPS.

It is an object of the present invention to provide a method for remotely monitoring a cardiac condition of an individual in which there is provided a two-way interactivity, since in addition to sending data of the patient being monitored to the SC - Service Center, at any given moment, the SC or a clinician may conditionally request a new exam on demand, thus allowing an actual and accurate tracking without the need of displacing the patient being monitored.

These and other objects which are not apparent will be described in detail in the description of the invention and its preferred embodiments.

### DESCRIPTION OF THE DRAWINGS

Figure 1 shows a schematic diagram of the method of the present invention;
Figure 2 shows a diagram integrating the routines carried out by the main module of the main branch of the system of the present invention;
Figure 3 shows a diagram integrating the modules of the main branch of the system of the present invention;
Figure 4 schematically shows an interface screen according to the present invention;
Figure 5 shows a schematic diagram of the apparatus of the present invention;
Figure 6 illustratively shows an arrangement form of the electrodes according to the present invention;
Figure 7 shows a wave pattern graph in a period of one ECG; and

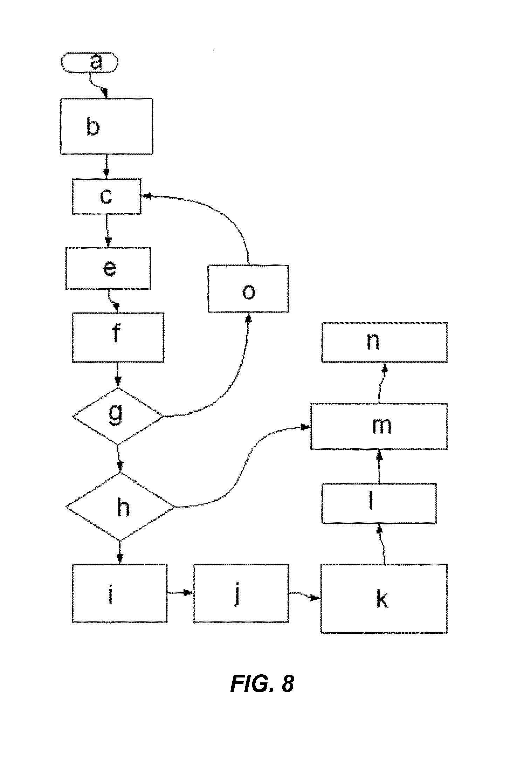

Figure 8 shows a flow diagram of the assistance routine of the cardiac monitoring of the present invention.

### DETAILED DESCRIPTION OF THE INVENTION

The present invention relates in a first aspect to a method for monitoring the cardiac condition of an individual in which there is provided a monitoring network which enables the continuous monitoring the cardiac condition of an individual allowing the patient and the clinician to remotely interact and providing the clinician with a tool for collecting, monitoring and viewing electrocardiograms in real time and over the Internet.

In this way, the patient will be able to have his/her cardiac condition analyzed by a clinician or medical staff at any time, wherever he/she is.

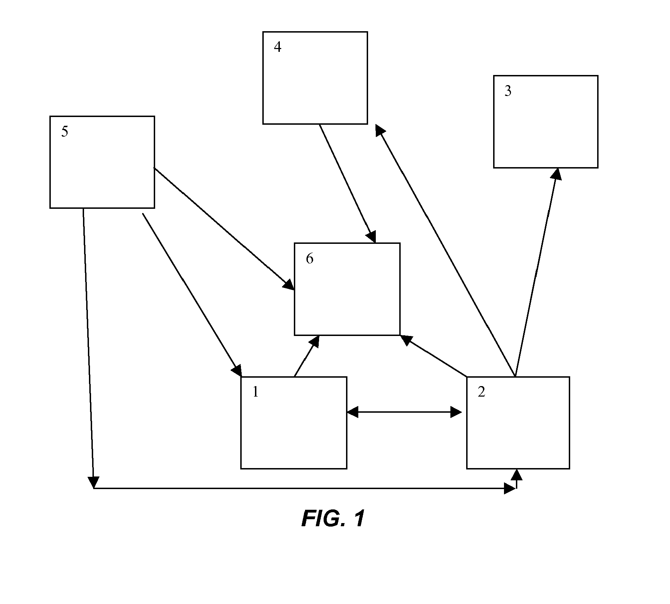

According to Figure 1, the method of the present invention comprises:
- Establishing a remote communication between an individual (1) whose cardiac condition is being monitored and a Monitoring Center (2);
- Establishing a remote communication between a Monitoring Center (2) and a Rescue/Assistance Unit (3);
- Establishing a remote communication between a Monitoring Center (2) and a Hospital Unit (4);
- Establishing a remote communication between an individual (1) whose cardiac condition is being monitored and a Medical Staff (5); and
- Establishing a Data Provision Interface (6) remotely accessible to registered users.

The remote communication, for example, between the individual (1) and the Monitoring Center (2) is established in a two-way fashion, i.e., both the individual (1) and the Monitoring Center (2) are able to mutually make requests such that the assistance and the update of data and information in real time are streamlined. The communication between the Individual (1) and the Monitoring Center (2) is performed by means of GSM and GPS communication. This enables an interaction between the individual (1) and the Monitoring Center (2) allowing requests to be readily forwarded as well as the individual (1) to be quickly positioned on an emergency situation for ready assistance.

ECG readings of the Individual (1) will be continuously carried out by means of software embodied into a remote monitoring apparatus (now shown), the detailed description of which will be provided hereinafter. In a critical situation, the reading which identified an irregularity at the cardiac condition of the individual will follow to the Monitoring Center (2) as an ECG exam together with further parameters which will be later defined in the present invention.

The ECG warning sent from the apparatus monitoring the Individual (1) will trigger in the Monitoring Center (2) a user assistance provision protocol even to the extent that a closest Rescue Unit (3) and a Hospital Unit (4) will be mobilized for ready assistance. The provision protocol will comprise a monitored profile of the Individual (1) and its location for rescue actions. Detailed information about the Individual (1) profile can be accessed on an Interface (6) updated in real time which will allow both the Rescue Unit (3) and the Hospital Unit (4) to speed up the medical assistance regarding the cardiac condition for the Individual (1) .

The Interface (6) can be accessed upon registration and allows access to profile information of the Individual (1) on emergency situations, but also for routine control. For example, an Individual (1) may have his/her records accessed by the Medical Staff in charge of following up that patient regularly, thus being possible to remotely follow up an administered treatment, a post-operative period, or even the reaction of the Individual to a new drug prescribed.

It is understood by "communication" in the method of the present invention the sending and reception of formal communications (via telephone networks), text and/or voice messages and data transfer via GSM communication. Among communications in the method of the present invention, only the communication between the Individual (1) and the Monitoring Center (2) is carried out in a two-way fashion, that is, the Individual (1) and the Monitoring Center (2) reciprocally receive requests via GSM as previously illustrated.

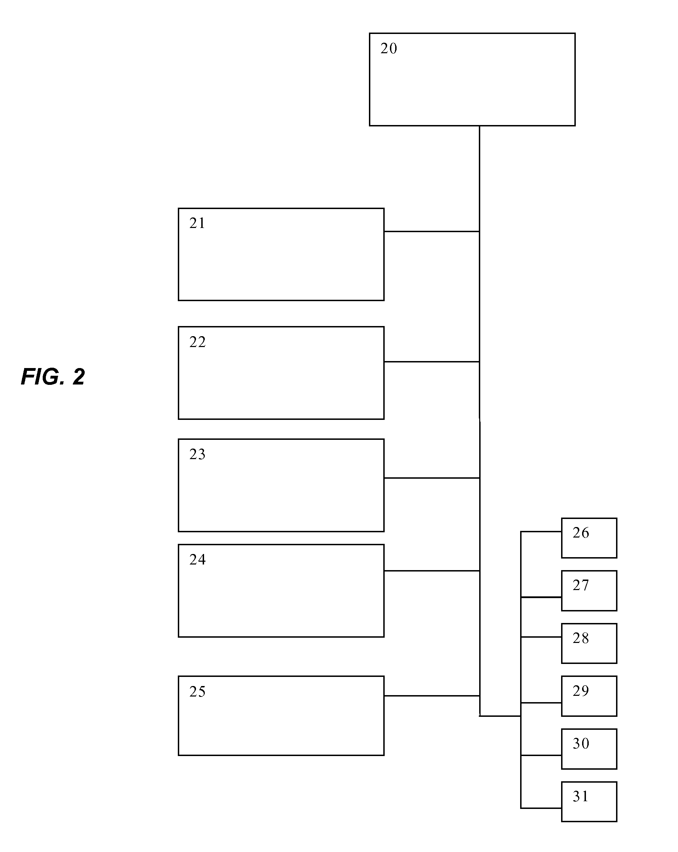

According to the present invention there is provided a system for remotely monitoring the cardiac condition of an individual. Typically, the system of the present invention comprises two module branches in which a main branch carries out the cardiac condition monitoring routines of the present invention and a managing branch carries out the routines interconnecting the network formed by the method of the present invention. The main branch comprises the following routine modules:
Main Module - Performs the activation functions of the apparatus of the present invention by carrying out the routines shown in Figure 2:
   - Initializing the environment and executing other modules (21);
   - Checking the SOS button - help button (22);
   - Checking the Battery levels (23);
   - Communication and information exchange between all the modules (24);
   - Requesting the execution of the ECG (25);
   - Requesting the file transfer to the data server (26);
   - Requesting the downloading of the file from the data server (27);
   - Requesting the sending of SMS (Short Message Service) - text message (28);
   - Requesting the monitor to vibrate as a warning means (29)
   - Requesting the buzzer to emit sound as a warning means (30);
   - Requesting an emergency call and/or sending SMS and location warning upon detecting a user fall (31)

ECG Module - the module responsible for generating ECG exams and checking cardiac intercurrences such as ischaemias or arrhythmias.

GSM Module - the module responsible for operating the communication via GSM/GPRS (Global System for Mobile Communications/General Packet Radio Service) and outputting sound through the buzzer. This module controls communications via hands-free phone calls and by sending and receiving SMSs. It is also responsible for transferring files and data from the monitor to the data server and vice versa. The SMSs received in encoded form, after being identified to the requested operations, are forwarded to the Main module which will take proper measures.

GPS Module - the module responsible for reading GPS (Global Positioning System) data from the monitor and reading the retrieved latitude and longitude coordinates. Those coordinates are forwarded to the Main module, which in turn forwards this information to the ECG module.

XYZ Module - the module responsible for reading and evaluating the coordinates of the accelerometer. This module detects when the apparatus moves or falls. By detecting a fall the XYZ module notifies the Main module to take the proper measures.

DNA Module - the module responsible for the PNN - Probabilistic Neural Network - for detecting intercurrences (arrhythmias and ischaemias). The most important property of neural networks lies in their ability to learn from the environment, thus improving their performance. This is made through a dynamic adjustment process applied to their weights on the training. The learning occurs when the neural network achieves a generalized solution for a class of problems. According to the present invention, the solution achieved by the neural network is a customized profile of the behavior pattern of the user's heart.

The term learning algorithm denotes a set of well-defined rules for solving a learning problem. There are many different types of learning algorithms specifically for given neural network models, and these algorithms differ from each other mostly in the way how weights are modified.

Another important factor is the way in that a neural network relates to the environment. In this context there are the following learning paradigms:
- Supervised Learning, when an external agent is used that indicates to the network the desired response for the input pattern;
- Non-supervised learning (self-organizing), when there is no external agent indicating the desired response for the input patterns.

It is meant by presentation cycle all N-couples (input and output) from the training set in the learning process. In the present invention, the neural network of the DNA Module has the non-supervised learning mode.

According to the present invention, the neural network of the DNA Module of the main branch has an Input Layer corresponding to the data collected from the Patient and an Output Layer corresponding to a menu containing categories of referential intercurrences.

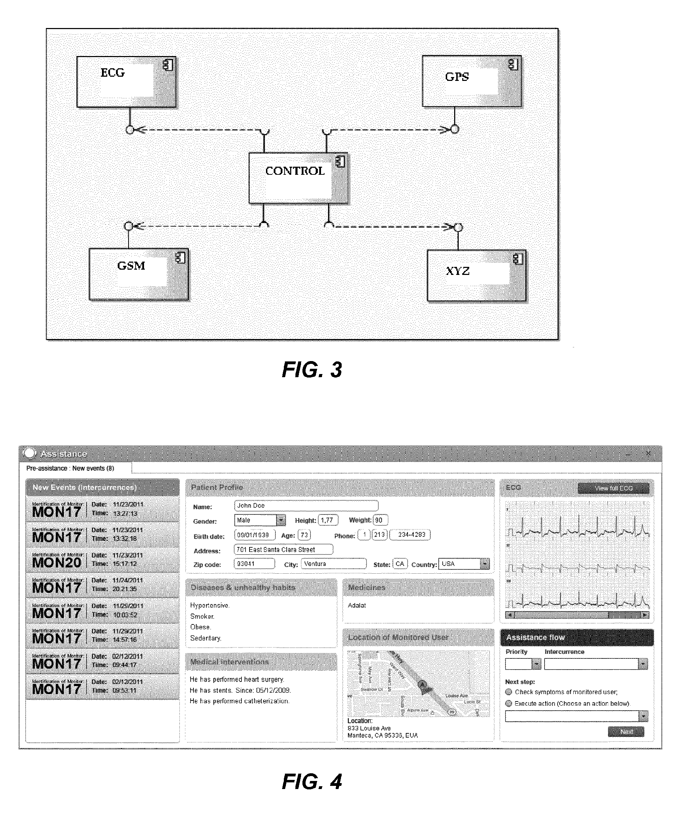

In Figure 3 it is shown a diagram integrating the modules of the main branch of the system of the present invention.

The managing branch comprises the following routine modules:
Monitoring Module - Comprises the routines for Client Assistance, Clinical Research and Patent Monitoring History;
Configuration Module - Comprises the routines for the classification of intercurrence types and intercurrence groups, routines of actions to be carried out, steps to be performed during assistance and a general symptoms record.
Network Module - Comprises the routines for user interaction, screens/modes and access profiles.
Medical Record Module - Comprises the routines for recording administered pharmaceuticals and medical specialties.
Product Module - Comprises the monitoring routines of the Monitoring Center.
Cooperative and Affiliated Members Register Module - Comprises the routines for personnel management and registration; Physicians, Health Entities, Healthcare Insurance Providers and Insurance Companies and Monitors and Clients.

Typically according to the present invention, the main branch acts embodied into a remote monitoring apparatus of the cardiac condition of an individual while the managing branch of the system of the present invention acts on the interfaces established from the method of the present invention. A nonlimiting example of the interface of the present invention is shown in Figure 4.

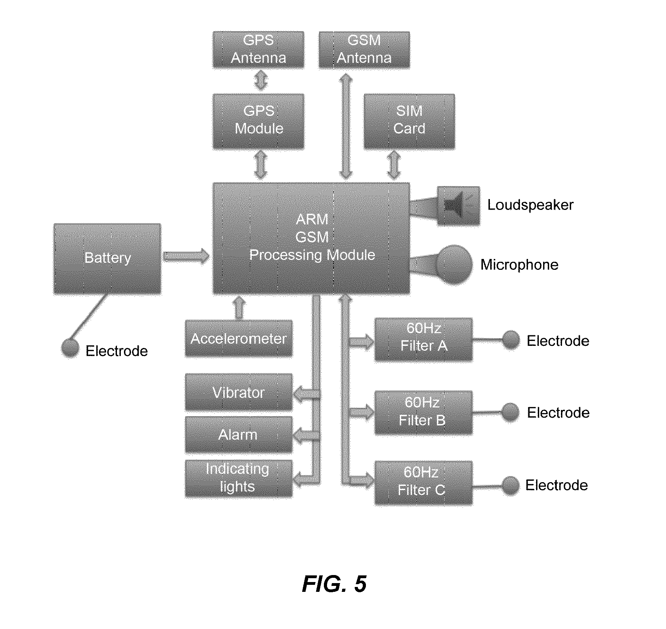

According to the present invention (Figure 5) there is provided an apparatus for remotely monitoring a cardiac condition of an individual.

The apparatus of the present invention allows to continuously pick up, analyze and monitor the behavior of the user's heart, such that, in case any intercurrence is detected, they are classified in two major groups: arrhythmias and ischaemias and being broken down into their subgroups, and to perform an immediate detection, generating an exam to be analyzed by a healthcare professional.

Thus, the apparatus has 4 (four) electrodes which allow the picking-up and behavior of the electrical cardiac impulses in three different forms - Derivations I, II and II and a fourth electrode which acts as the ground, reducing noises and interferences with the electrical signals received. For the medical literature, those derivations are the most complete and enable a comprehensive view of the human heart from several angles. The electric signals from the heart are picked up analogically, amplified and converted from the analog format into the digital format. Once in this format, those data are processed on an internal microprocessor which compare them with a database based on 'neural networks. Upon detecting any difference between the picked up plotting and the recognized database, the apparatus automatically generates an ECG exam, which is transformed into a data packet to be sent via cellular network (GSM/GPRS) to a Monitoring Center for analysis.

Additionally the apparatus of the present invention comprises a GSM antenna designed in the vertically polarized dipole system; a GPS antenna designed in the horizontally polarized helical system; a hands-free set provided with an omni-directional electret microphone; a loudspeaker; a plug for connecting the electrode cables to the monitor being of the P2 stereo type with microphone; a set of electrode cables; A tact-switch type button; a side volume control; and circuits with diodes and transistors.

The apparatus is also provided with a hands-free system such that the Monitoring Center may contact the user and vice-versa and the healthcare professionals are able to collect further information from the user and indicate the best measure for user assistance or, in case the user is unable to move, dispatching immediately a rescue unit.

The apparatus of the present invention also has an Accelerometer device which is able to identify user falls, a common symptom after a severe heart attack, therefore an ECG received related to a patient fall requires a still more urgent assistance.

In order to make easier the localization and guiding of the user to the nearest Health Center, the apparatus has a GPS-assisted localization system able to provide the location - Latitude and Longitude - to the user and correlate it with maps and hospital networks to readily assist the person.

Since it is connected to the cellular network, the apparatus can be handled remotely via software, which allows a remote exam to be generated by a clinician, thus changing the way exams are made in that it no longer depends on the person's will, making the data collection possible at any time and at any place having cellular network coverage.

The apparatus of the present invention has the monitoring function and provides a series of benefits over conventional technologies such as: embodied intelligence, without the need of surgical intervention for installation, improved autonomy, ergonomics and portability.

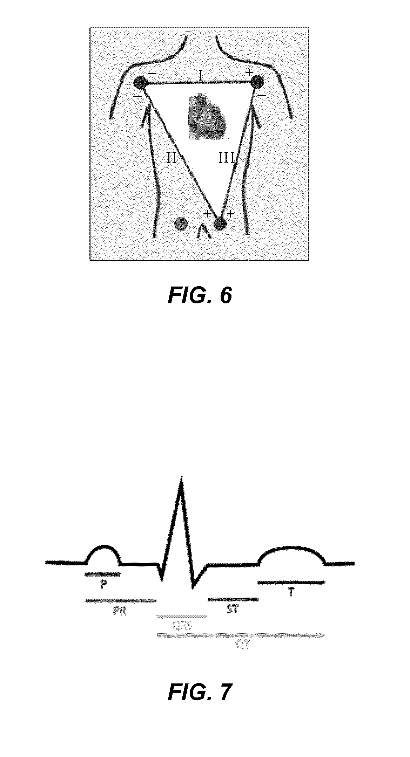

According to the present invention the apparatus is typically portable, mobile, non-invasive, including hardware, software and a back-office application allowing for the smart and autonomous remote monitoring - via GSM/GPRS, detecting cardiac intercurrences (arrhythmias and ischaemias) through 3 electrodes (Figure 6, derivations I, II and III), falls, as well as integrating hands-free assistance and geographical localization via GPS and allowing to track the monitoring via Internet.

The solution may be described in a "two-way" fashion, since in addition to sending data from the monitored patient to the SC - Support Center, a clinician or the SC may conditionally request, on demand, a new exam at any time.

The GSM antenna is designed in the vertically polarized dipole system. The GPS antenna is designed in the horizontally polarized helical system.

For consumption management purposes, the system is provided with circuits having diodes and transistors for potentially different situations and battery charge saving.

### DESCRIPTION OF THE PREFERRED EXEMPLARY EMBODIMENT

### OF THE INVENTION

In the following, an exemplary embodiment of the present invention will be provided. Although certain modifications and variations will become apparent from the present description, such modifications and variations are comprised within the scope of the present invention.

### CARDIAC MONITORING

Electrocardiogram (ECG) consists in recording the electrical phenomena which originate during the cardiac activity by means of an apparatus called electrocardiograph. The electrocardiograph is a galvanometer (an apparatus that measures the potential difference between two points) which measures small current intensities collected from two electrodes (small metal plates connected to a conducting wire) arranged on determined points of the human body. It serves as a valuable aid in the diagnostic of a large number of cardiopathies and other conditions such as, for example, hydroelectrolytic disturbances.

The potential difference between two members was introduced by Einthoven, which conceived the heart in the center of an equilateral triangle whose vertices would be represented by the right arm (R), the left arm (L), and the left leg (F). The positioning of electrodes and bipolar derivations are made according to Einthoven's triangle (Figure 6). This orientation was based on Kirchhoff second law, which states that in a closed loop, the sum of potential differences equals to zero. In this triangle, Einthoven inverted the polarity of DII in order to obtain a positive record of the wave R in the three derivations.

The connections made are:
- DI=VL-VR (left arm - right arm)
- DII=VF-VR (left leg - right arm)
- DIII=VF-VL (left leg - left arm)

The ECG exam is indicated as part of the analysis of heart disease, particularly cardiac arrhythmias and ischaemias. ECG is useful in the diagnostics of acute myocardial infarction, being the preferably adopted exam on emergencies together with the dosage of cardiac enzymes.

This record generated by the ECG exam shows the electric potential variation in time, generating a linear image, on waves. These waves follow a rhythmic pattern having a particular terminology.
- P wave: corresponds to the atrial depolarization.
- QRS complex: corresponds to ventricular depolarization; it is higher than P wave since the muscle mass of the ventricles is greater than that of atria.
- T wave: corresponds to ventricular repolarization; the reversal of the T wave indicates an ischemic process.

Upon receiving electric signals from the human body, ranging from 1mV to 5mV, these are collected by 3 electrodes arranged as described above. These impulses - having a sampling rate of 360 Hz - are amplified up to 1000 times by means of an operational amplifier. Thereafter, a series of filters are applied to remove interfering noises.

The aim is to remove through successive filtrations the 60 Hz noise from the power grid, the muscle noise (EMG - Electromyogram), and the baseline wander.

In order to remove the noise from the electrical grid, as well as the muscle noise, a Butterworth low-pass band filter having a rejection range (cut frequency) of 40 Hz is used.

For removing the baseline wander a Butterworth high-pass band filter having a rejection range (cut frequency) of 0,67Hz is used.

After filtering, indicators (amplitude and time) of the relevant characteristics of the ECG, such as: P wave, QRS complex, T wave, SR segment, RR interval, etc. are extracted and normalized, thus forming a vector to be presented to the input layer of the RNP.

After processing in parallel - verifying a set of previously stored vectors - the RNP decision layer (basing upon an algorithm similar to k-nearest neighbor) selects the class (c) that best meets the characteristics shown.

Initially, numerous vector models (based on the database of arrhythmias and ischaemias of MIT-BIH) are stored in the RNP - respectively with the values from the output layer, which form the classes (clusters) - relating to expected results: normal, arrhythmia, ischemia, etc.

Having detected an intercurrence (arrhythmia or ischemia), the 10 seconds during the cardiac event that occurred are stored in a physical file in binary format. These files are kept in a specific directory and can be subsequently retrieved or transferred once again.

Together with data relating to the intercurrence, relevant information is added such as: geographical location of the monitored individual obtained through the longitude and latitude coordinates obtained via GPS (Global Positioning System).

The transmission of these data will be made through the GPRS (General Packet Radio Service) services network which is made available through the GSM (Global System Mobile) connection provided by cell phone signal. For data transmission, the FTP (File Transfer Protocol) is used which allows the transfer from compressed and partitioned files in packets of 256 bytes, from the NEXCOR monitor to the remote data server.

Data compression is performed by using a Linux application known as "gzip". This application compresses using an algorithm based on the public domain Lempel-Ziv code. The data compression is important since:
- It permits to reduce the size of data files, enabling the storage of a greater amount of information;
- The transfer of smaller files will be carried out much faster; as the data transfer rate is limited in the GSM/GPRS technology, this compression enables to reduce costs in using the data bandwidth.

The fall detection is another process being executed in parallel; for this purpose an accelerometer is used, which is a system able to measure the acceleration of a movement in a three-dimensional axis. By reading these variations it is possible to detect movements - aiding in filtering and removing the artifact motion from the ECG, which interfere with the plotting - as well in identifying falls, whether resulting from an cardiac intercurrence or not. The falls are also monitored and sent to the Support Center, together with the location via GPS and an ECG exam.

The battery consumption is also monitored in a parallel process, wherein upon reaching a critical level the user is warned - via leds and vibration - of the current condition, thus suggesting an immediate substitution.

The support to the coverage of the GSM cellular network is also monitored, wherein the user is warned - via leds and sound signal - of the current condition. This process involves, upon re-establishing the connection, sending various information, such as: ECG, location, etc. to the Support Center.

At any time the Support Center or the user may establish contact via hands-free in order to dispel doubts or request assistance.

The on-demand request, made from the Support Center or conditionally by a clinician, requests once again an exam allowing to confirm the received exam - which originally raised doubts. This event may be triggered from the back-office application of the SC, or by means of a simple SMS from a cell phone.

A specific example of the cardiac monitoring of the present invention can be seen in Figure 9: Initially during the routine monitoring of an individual (a), the monitor detects an irregular heart beating (b); then the monitor generates an ECG (c) and sends it to the Cardiac Monitoring Center (d). A clinician in the Cardiac Monitoring Center carries out an analysis on the received ECG (e) and the clinician may then decide (f) to request a new ECG (o) or check the symptoms (g, h). The clinician may decide to initiate an assistance action and request the attendant in the Cardiac Monitoring Center to initiate an assistance action (m, n). If the clinician chooses to check the individual's symptoms, he/she may request an attendant to remotely check (i, j) the symptoms; after checking, the attendant sends a new ECG with the symptoms for analysis by the clinician (k, 1) and from this action an assistance action may be initiated (m, n).

It is also possible to carry out the remote update of the monitoring module of the apparatus of the present invention through the OTA (Over The Air) concept by simply sending a SMS containing specific commands for this case. That is, in case of updating the firmware or software of the monitor itself, the user does not need to be displaced.

## Claims

1. A method for remotely monitoring a cardiac condition of an individual, the method being **characterized in that** it comprises:
• establishing a remote communication between an individual (1) whose cardiac condition is being monitored and a Monitoring Center (2);
• establishing a remote communication between a Monitoring Center (2) and a Rescue/Assistance Unit (3);
• establishing a remote communication between a Monitoring Center (2) and a Hospital Unit (4);
• establishing a remote communication between an individual (1) whose cardiac condition is being monitored and a Medical Staff (5); and
• establishing a Data Provision Interface (6) remotely accessible to registered users.

2. Method, according to claim 1, **characterized in that** it comprises:
a remote communication established in a two-way fashion wherein requests may be reciprocally made to transmit data from the individual (1) or the Monitoring Center (2) to the Individual (1).

3. Method, according to claim 2, **characterized by** comprising a GSM remote communication.

4. Method, according to claim 1, **characterized by** comprising a remote monitoring in which falls suffered by the Individual (1) are also monitored and sent to the Monitoring Center (2), together with the location via GPS and an ECG exam.

5. Method, according to claim 1, **characterized by** continuously reading the ECG of the individual (1) by means of the remote intercurrence monitoring apparatus.

6. Method, according to claim 1, **characterized in that** a provision protocol triggered by the detection of an intercurrence comprises a monitored profile of the Individual (1) and his/her location.

7. Method, according to claim 1, **characterized by** comprising an Interface (6) accessible through registration and which allows access to profile information of the Individual (1) for emergency situations and routine control.

8. A system for remotely monitoring the cardiac condition of an individual **characterized by** a main branch which comprises:
• a Main Module that performs the activation functions of a apparatus for monitoring the cardiac condition of an individual;
• an ECG Module that carries out the ECG exams and checks cardiac intercurrences;
• a GSM Module that performs the operation of the communication via GSM/GPRS and outputting sound through the buzzer;
• a GPS Module responsible for reading GPS data from the monitor and retrieving latitude and longitude coordinates;
• a XYZ Module that reads and evaluates the coordinates of an accelerometer;
• A DNA Module for detecting intercurrences by means of a Probabilistic Neural Network.

9. System, according to claim 8, **characterized by** comprising a Probabilistic Neural Network that operates in a non-supervised mode.

10. System, according to claim 8, **characterized by** comprising a Probabilistic Neural Network in which an Input Layer comprises the ECG data of the patient and the Output Layer comprises categories of cardiac intercurrences.

11. System, according to claim 10, **characterized by** comprising a solution achieved by the Probabilistic Neural Network that comprises a customized profile of an individual.

12. System, according to claim 8, **characterized by** comprising a Main module which performs the routines of:
• initializing the environment and executing the other modules;
• checking the SOS button - help button;
• checking the battery levels;
• communicating and exchanging information between all the modules;
• requesting the execution of the ECG (Electrocardiogram);
• requesting the file transfer to the data server; requesting to download the file from the data server;
• requesting the sending of a SMS (Short Message Service) - text message;
• requesting the monitor to vibrate as a warning means;
• requesting the buzzer to emit sound as a warning means; and
• requesting an emergency call and/or sending an SMS and location warning upon detecting a user's fall.

13. System, according to claim 8, **characterized by** comprising an ECG module that answer the requests from the Main module with respect to the ECG execution and reading of battery levels.

14. System, according to claim 8, **characterized by** comprising a GSM module which controls communications by means of hands-free phone calling and by sending and receiving SMSs.

15. System, according to claim 8, **characterized by** comprising a GSM module which mutually performs the transfer of files and data from the monitor to the data server and the SMS communications received in encoded form are forwarded to the Main module which will take proper measures after the requested operations are identified.

16. System, according to claim 8, **characterized by** comprising a GPS module in which coordinates are transmitted to the Main module which forwards this information to the ECG module.

17. System, according to claim 8, **characterized by** comprising a module XYZ which detects when the apparatus is moves or falls and notifies the Main module to take the proper measures.

18. System for remotely monitoring a cardiac condition, **characterized by** a managing branch comprising:
• a monitoring Module comprising routines for the Client Assistance, Clinical Research and Patent Monitoring History;
• a Configuration Module comprising routines for the classification of intercurrence types and intercurrence groups, routines of actions to be carried out, steps to be performed during the assistance and a general symptoms record;
• a Network Module comprising routines for user interaction, screens/modes and access profiles;
• a Medical Record Module comprising routines for recording administered pharmaceuticals and medical specialties;
• a Product Module comprising the monitoring routines of the Monitoring Center;
• a Cooperative and Affiliated Members Register Module comprising the routines for personnel management and registration: Physicians, Health Entities, Healthcare Insurance Providers and Insurance Companies and Monitors and Clients.

19. System, according to claims 8 and 18, **characterized in that** a main branch acts embodied into a remote monitoring apparatus of the cardiac condition of an individual while the managing branch acts on the established interfaces.

20. System, according to claim 19, **characterized in that** the remote monitoring of an intercurrence comprises the cardiac condition of an individual selected from one of arrhythmia and ischaemia.

21. An apparatus for remotely monitoring a cardiac condition of an individual, **characterized in that** it comprises a GSM antenna designed in the vertically polarized dipole system; a GPS antenna designed in the horizontally polarized helical system; a hands-free set provided with an omni-directional electret microphone; a loudspeaker; a plug for connecting the electrode cables to the monitor being of the P2 stereo type with microphone; a set of electrode cables; A tact-switch type button; a side volume control; and circuits with diodes and transistors.

22. Apparatus, according to claim 21, **characterized by** comprising the autonomous and smart remote monitoring via GSM/GPRS of cardiac intercurrences, falls, integrated hands-free support and geographical localization via GPS.

23. Apparatus, according to claim 21, **characterized by** comprising the monitoring of arrhythmias and ischaemias.

24. Apparatus, according to claim 21, **characterized by** comprising a remote autonomous and smart monitoring by means of a two-way communication.
